(19) Europäisches Patentamt
European Patent Office
Office européen des brevets

(11) **EP 2 500 724 B1**

(12) **EUROPEAN PATENT SPECIFICATION**

(45) Date of publication and mention
of the grant of the patent:
**30.04.2014 Bulletin 2014/18**

(51) Int Cl.:
***G01N 33/12*** *(2006.01)*     ***G01N 33/10*** *(2006.01)*
***A23L 1/00*** *(2006.01)*     ***G01R 27/02*** *(2006.01)*

(21) Application number: **11158699.6**

(22) Date of filing: **17.03.2011**

(54) **A method for obtaining information on food stuff in or for a cooking process**

Verfahren zur Informationsgewinnung über Lebensmittel in einem oder für einen Kochprozess

Procédé pour obtenir des informations sur des aliments dans ou pour un processus de cuisson

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**19.09.2012 Bulletin 2012/38**

(73) Proprietor: **Electrolux Home Products Corporation N.V.**
**1130 Brussel (BE)**

(72) Inventors:
• **Luckhardt, Christoph**
**91607 Gebsattel (DE)**
• **Ruther, Florian**
**91628 Steinsfeld (DE)**

(74) Representative: **Röder, Richard**
**Electrolux Dienstleistungs GmbH**
**Group Intellectual Property**
**Fürther Strasse 246**
**90327 Nürnberg (DE)**

(56) References cited:
**EP-A1- 1 253 382**    **EP-A2- 0 301 699**
**EP-A2- 0 375 366**    **EP-A2- 2 026 632**
**WO-A1-99/01754**    **WO-A1-2008/147988**
**DE-A1- 3 621 999**    **GB-A- 2 298 923**
**US-A1- 2010 086 655**    **US-A1- 2011 052 767**

• **TROELTZSCH U ET AL: "Untersuchungen zur Machbarkeit von Fleischqualitaetsbewertung mit Impedanzspektroskopie", SENSOREN UND MESSSYSTEME 2010 : VORTRÄGE DER 15. ITG/GMA-FACHTAGUNG VOM 18. BIS 19. MAI 2010 IN NÜRNBERG, FACHTAGUNG SENSOREN UND MESSSYSTEME <15, 2010, NÜRNBERG> INFORMATIONSTECHNISCHE GESELLSCHAFT, DE, 18 May 2010 (2010-05-18), pages 134-139, XP008148790, ISBN: 978-3-8007-3260-9**
• **YUTAKA KITAMURA ET AL: "Electric Impedance Spectroscopy for Yogurt Processing.", FOOD SCIENCE AND TECHNOLOGY RESEARCH, vol. 6, no. 4, 1 January 2000 (2000-01-01) , pages 310-313, XP055019526, ISSN: 1344-6606, DOI: 10.3136/fstr.6.310**
• **D Hardy ET AL: "Rapid detection of microbial contamination in frozen vegetables by automated impedance measurements.", Appl. Environ. Microbiol, vol. 34, no. 1 1 January 1977 (1977-01-01), pages 14-17, XP055020639, Retrieved from the Internet: URL:http: //aem.asm.org/content/34/1/14.ful l.pdf#page=1&view=FitH [retrieved on 2012-02-29]**
• **Food and Agriculture Organization of the United Nations (FAO): "Section 3 - The Hazard Analysis and Critical Control Point (HACCP) System", Food quality and safety system. A Training Manual on Food Hygiene and the Hazard Analysis and Critical Control Point (HACCP) System , 1998, pages 1-80, XP002670514, Retrieved from the Internet: URL:http://www.fao.org/ docrep/W8088E/w8088 e05.htm#section 3 [retrieved on 2012-03-05]**

EP 2 500 724 B1

**(Cont. next page)**

- J Niu ET AL: "A new approach for the determination of fish freshness by electrochemical impedance spectroscopy", Journal of Food Science, vol. 65, no. 5 1 January 2000 (2000-01-01), XP055020641, Retrieved from the Internet: URL:http://lib3.dss.go.th/fulltext/ Journal /Journal of food science/2000 v.65/no. 5/jfsv65n5p0780-0785ms19990847[1]. pdf [retrieved on 2012-02-29]

- J Niu ET AL: "A new approach for the determination of fish freshness by electrochemical impedance spectroscopy",

**Description**

The present invention relates to a method for obtaining information on food stuff in or for a cooking process method. Further, the present invention relates to a food probe.

The cooking process of substantially homogenous food stuffs like meat, potatoes and other vegetables, pies and some casseroles is monitored mainly by detecting the core temperature of the food stuff. The user has to know at which core temperatures the food stuff has certain desired properties like colour, tenderness or degree of cooking.

In order to simplify the cooking process for the user, automatic cooking functions are available. Said cooking functions are based on an information input from the user and/or measurements by sensors such as temperature probes. The determination of food properties by measurements allows a reduction of the information input from the user.

The detection of the electrical impedance of the food stuff can provide a lot of information for the automatic cooking functions.

US 2006/0174775 A1 discloses a method and an apparatus for tracing a cooking process. At least two separated electrodes are inserted into the food stuff. The electrical impedance is measure at a certain frequency in order to monitor the cooking process.

DE 36 21 999 A1 discloses a method for monitoring a cooking process of food. An elongated food probe with at least two electrodes in the front portion is inserted into the food stuff. An electrical impedance of the food stuff is detected in the beginning of the cooking process and during the further cooking process.

EP 0 375 366 discloses a method and a food probe for determining food type and properties from measured impedance values.

It is an object of the present invention to provide a new method for obtaining information on food stuff in or for a cooking process.

This object is achieved by the method according to claim 1. Further embodiments and improvements can be obtained from the dependent claims.

In a preferred embodiment at least one electric voltage (or: electric field) in general having AC components or being an AC voltage is applied to said region of the food stuff and the impedance or the at least one component thereof in that region of the food stuff is measured or detected. At least one food probe is used which is inserted into the food stuff and comprises at least two electrodes for applying the electric voltage and measuring the impedance or the at least one component thereof. The impedance can for instance be measured or determined by measuring the electric voltage and the electric current in the region of the food stuff, in particular between the electrodes, which gives information on the impedance, including the phase angle between voltage and current. Of course measuring the impedance also includes using the measured values of the voltage and current directly, as they are unambiguously related to the impedance, without an intermediate calculation of the impedance.

The at least one electrical component of the electrical impedance is in particular chosen from the group comprising the ohmic resistance, the reactance, the capacity, the inductivity, the modulus and the phase angle.

During the step of deriving information about the at least one characteristic property of the food stuff from said detected impedance or component thereof in one variant according to the invention a comparison is made with stored pre-determined or empirically determined reference data for the characteristic property of the food stuff, including for instance meat and/or fish and/or vegetables, in particular stored in a reference data base and/or as reference data sets, e.g. a first set of reference data for the food type and a second set of reference data for the food age.

The deriving or determining of the food type is preferably made or performed before the deriving of information about the age or freshness of the food stuff and/or preferably based on the same detected impedance or component values as with the deriving information about the age or freshness of the food stuff. This means that the detection or measurement of impedance or component thereof has to be made only once and then firstly the food type and then with the information on the food type, the age or freshness of the food stuff of this food type is determined, wherein only a set of reference data for only this food type has to be accessed. This can apply also to other food properties. But it is also possible to make separate measurements for type of food stuff and age of foodstuff or other characteristic property respectively.

The reference data supplied by the manufacturer can be supplemented during use by the user who can add other food types or food age or other characteristic food criteria through an input device.

In a preferred embodiment the electrical impedance or the at least one component thereof of the food stuff for deriving of information on the characteristic property of the food stuff is detected before or at the beginning of the cooking process and/or at a predefined, preferably low, temperature e.g. room temperature, or in a predefined temperature range, preferably low, temperature e.g. room temperature. This way the influence of temperature on the measurements is greatly reduced.

Furthermore the electrical impedance or the at least one component thereof of the food stuff for a subsequent deriving of information on the characteristic property of the food stuff can be measured several times in a pre-given time interval, e.g. several minutes, at pre-determined instants of time, e.g. every 10 to 60 seconds, to reduce measuring errors or get additional information about the food stuff.

In a further advantageous embodiment the information about the characteristic property, in particular food type and/or the age or freshness, of the food stuff is used for controlling or adapting the cooking time and/or cooking temperature of the cooking process dependent on this information about the age or freshness. For instance, for less fresh or aged food stuff or for food stuff that was frozen the cooking time is selected to be longer and/or the cooking temperature is selected to be higher or to be at a high level for a longer time.

According to the invention, the deriving (or: recognition or determining) of the information on the characteristic property of the food stuff is done based on the measurement or detection made at two or even more (n > 1) frequencies, in particular of the electric voltage applied, which allows for a clear and in all case unambiguous recognition of the characteristic property of the food. Preferably n is 2, 3, 4 or 5 without limitation.

In particular, for n > 1 frequencies, m measuring steps are performed with $0 < m \leq n$ wherein in each measuring step a voltage having at least one of the n frequencies is applied and wherein each of the n frequencies is contained in at least one of the applied m voltages. For instance, there can be a measurement with n measuring steps in which several voltages of a single frequency are applied one after the other wherein the frequency of the voltage is varied or changed through the pre-set n > 1 frequency values. Alternatively, a voltage having all n > 1 frequencies can be used in a single measurement combined with a frequency analysis. Also, any combination can be used, e.g. for n frequencies a measurement with a voltage with n - m frequencies with m < n combined with m measurements with a voltage of a single frequency out of the remaining m frequencies.

When two different frequencies are used, two reasonable frequencies are 50 kHz and 5 kHz, although there is no limitation whatsoever to these specific values.

The values of the impedance or component thereof detected at the one, two or more frequencies are in a preferred embodiment compared with the same number of stored reference values for characteristic property, in particular food types and/or age or freshness, of food stuff previously determined at the same frequencies.

The characteristic property, in particular food type or food stuff age or freshness, is derived in particular by determining the highest degree of coincidence with the reference values, e.g. by some mathematical norm, for instance Euklidian norm, or metric, or by using a function, e.g. a fit function or a function mapping the n values of the impedance or its component to a calculated function value, of the detected values of the impedance or component thereof at the two or more frequencies.

In other variants, the information on the characteristic property, in particular type or age, of the food stuff can be derived from a ratio or a difference or sum or even a ratio of a difference and the sum of two detected values of the impedance or component thereof at two different frequencies.

In one embodiment the ohmic resistance is used as the component of the impedance to derive information on characteristic property, in particular type or age, of the food stuff, as the ohmic resistance differs over a same frequency spectrum for different characteristic properties, in particular for different food types as well as for fresh food stuff as compared to aged food stuff, in absolute values as well as in the first derivative and the second derivative. In particular, at lower frequencies, the ohmic resistance of fresh food stuff, in particular meat such as poultry, is higher than that of aged food stuff. Further the ohmic resistance of fresh food stuff decreases more steeply with increasing frequency than that of aged food stuff. Also, at high frequencies, the ohmic resistance of the fresh food stuff is higher than that of aged food stuff. It can even be evaluated that the curve of the ohmic resistance for fresh food stuff can have a turning point in contrast to aged food stuff.

In an alternative embodiment the reactance is used as the component of the impedance to derive information on the characteristic property, in particular type or age, of the food stuff, as also the reactance differs over a same frequency spectrum for a different characteristic property, in particular for different food types as well as for different food age, in absolute values as well as in the first derivative and the second derivative. For instance, the reactance of fresh food stuff, in particular meat such as poultry, as well as of aged food stuff has a minimum in a given frequency spectrum and the reactance of the fresh food stuff at the minimum is smaller than the reactance of the aged food stuff at the minimum.

In yet another embodiment the phase angle is used as the component of the impedance to derive information on characteristic property, in particular type or age, of the food stuff, as also the phase angle differs over a same frequency spectrum for a different characteristic property, in particular for different food types as well as for different food age, in absolute values as well as in the first derivative and the second derivative. For instance, the phase angle of fresh food stuff, in particular meat such as poultry, has a minimum in a given frequency spectrum whereas the phase angle of aged food stuff has no minimum in this given frequency spectrum. Also the ratio of phases angles at two different frequencies decreases with increasing age of the food stuff and is in particular used to check the quality of the food stuff before or at the beginning of the cooking process.

The deriving from said detected impedance or component thereof information about the characteristic property, in particular type and/or age or freshness, of the food stuff can be performed in particular by calculating one or more electrical parameters of the food stuff from the electrical impedance or component thereof and comparing said electrical parameters with a data base.

Furthermore, in a specific embodiment, the electrical impedance or the at least one component thereof of the food stuff is detected again in another step during the cooking process and/or when the food stuff has been subjected to cooking already, for determining the cooking progress and/or cooking temperature in the region of the food stuff. In particular a ratio of phase angles at two different frequencies is calculated as a function of the temperature of the food stuff.

In a specific embodiment it is also possible to derive further to or as the information about the age or freshness of the food stuff an information on the estimated amount or concentration of bacteria in the food stuff.

In all embodiments it is also possible to derive information on the content of fat of the food stuff and/or whether the food stuff had been frozen before or not, in addition to the food type and the age or freshness of the food stuff.

Also the method and food probe according to the invention can in all embodiments be combined with temperature measurements using separate temperature sensors to obtain further information about the food stuff and cooking process.

The invention will be described in further detail with reference to the drawings, in which

FIG 1 illustrates a schematic view of a food probe according to a preferred embodiment of the present invention,

FIG 2 illustrates a schematic diagram of a phase angle as a function of the frequency for several types of food according to the preferred embodiment of the invention,

FIG 3 illustrates a schematic diagram of a ratio of two phase angles at different frequencies as a function of the temperature according to the preferred embodiment of the invention,

FIG 4 illustrates a schematic diagram of an ohmic resistance as a function of the frequency for poultry of different age according to the preferred embodiment of the present invention,

FIG 5 illustrates a schematic diagram of a reactance as a function of the frequency for poultry of different age according to the preferred embodiment of the present invention,

FIG 6 illustrates a schematic diagram of a phase angle as a function of the frequency for poultry of different ages according to the preferred embodiment of the present invention,

FIG 7 illustrates a schematic diagram of the ratio of two phase angles at different frequencies as a function of the age of the food stuff according to the preferred embodiment of the present invention, and

FIG 8 illustrates a schematic diagram of a number of bacteria as a function of the age of the food stuff according to the preferred embodiment of the present invention.

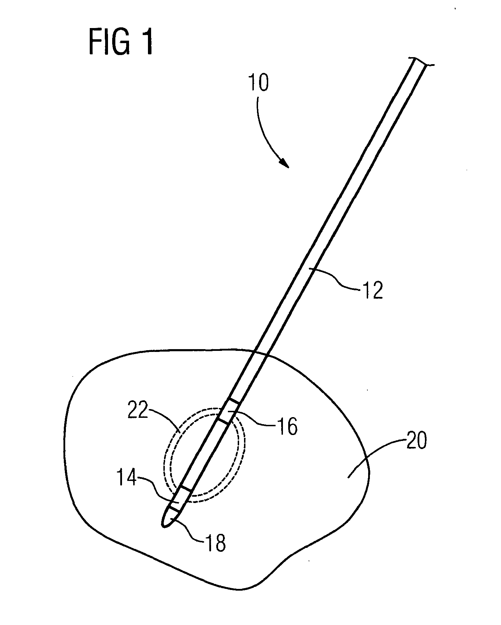

FIG 1 illustrates a schematic view of a food probe 10 according to a preferred embodiment of the present invention. The food probe 10 is provided for recognizing the type and/or characteristics of food stuff and monitoring a cooking process of such food stuff in a cooking oven or a cooking vessel.

The food probe 10 comprises an elongated rod 12, a first electrode 14, a second electrode 16 and a spike 18. A front portion of the food probe 10 is penetrated or inserted into food stuff 20.

The rod 12 is made of a non-conductive material. The first electrode 14 and the second electrode 16 are made of a conductive material. The spike 18 is made of a non-conductive material again.

The spike 18 may be made of the same non-conductive material as the rod 12 or by another non-conductive material. Alternatively, the spike 18 may be made of a conductive material.

The spike 18 is arranged at a front end of the rod 10. The spike 18 allows that the food probe 10 can easily be inserted or penetrated into the food stuff 20.

The first electrode 14 and the second electrode 16 are also arranged within the front portion of the rod 12. The first electrode 14 is arranged besides the spike 18. The second electrode 16 is also arranged within the front portion of the rod 12, but in a predetermined distance from the first electrode 14. Thus, the first electrode 14 and the second electrode 16 are electrically isolated from each other.

When the front portion of the food probe 10 is inside the food stuff 20 in an inner region thereof, then the first electrode 14 and the second electrode 16 are also arranged within the food stuff 20.

When a voltage is applied between the first electrode 14 and the second electrode 16, an electric voltage 22 is generated within the food stuff 20 in its inner region. Said electric voltage 22 extends between and in the environment of the first electrode 14 and the second electrode 16 and usually at least contains AC components or is a AC voltage.

Preferably, the front portion of the food probe 10 is inserted into the food stuff 20 in such a way, that the electric voltage 22 is generated within the central portion of the food stuff 20.

The serially arranged electrodes at the food probe 10 allow the generation of the electrical voltage 22 in the core or a central inner region of the food stuff 20. The food probe 10 is a compact tool and easy to handle.

The food probe 10 is provided for probing the food stuff 20 and in particular for detecting an electrical impedance Z of the food stuff 20 in an inner region thereof by applying the electric voltage 22 between the electrodes 14 and 16 in this region and measuring the impedance Z or an electrical component thereof between the electrodes 14 and 16.

The impedance Z or an electrical component thereof can for instance be measured or determined by measuring the electric voltage 22 and the electric current in the region of the food stuff, in particular between the electrodes and/or by means of LCR metering, and using basically the complex number relation between complex voltage U and complex current I with the complex impedance Z:

$$U(t) = Z(t) * I(t)$$

So, in all embodiments, when the detecting of impedance Z and components thereof and information derived thereof is mentioned this of course also includes using the measured values of voltage and current directly as these are measured values representing the impedance and components thereof or being unambiguously related thereto.

An evaluation unit, which is in particular part of or integrated in a control unit, for instance a microprocessor with corresponding storage,(none shown) now evaluates the influence the food stuff 20 has on this measured impedance Z, in particular at two or more frequencies, and preferably measured in the beginning of the cooking process and during the further cooking process. The evaluation or control unit is electrically connected with both electrodes 14 and 16 of the food probe 10 and provides the electrical voltage for the measurement and measures the (drop of the) voltage and/or the current over time or, alternatively, frequency between the electrodes 14 and 16.

The detected values of the electrical impedance Z may be compared with a reference data base, which is integrated in or accessed by the evaluation or control unit, in order to determine the type and/or characteristics of food. This will be explained in more detail in the following.

The electrical impedance Z is the electrical resistance for DC and/or AC currents and mathematically it is a complex number which can be represented by

$$Z = Re(Z) + i*Im(Z) = R + i*X \qquad\qquad (1)$$

including the imaginary unit i.

The ohmic resistance (or: DC resistance) R is the real part Re(Z) of the electrical impedance Z.

The reactance (or: AC resistance) X is the imaginary part Im(Z) of the electrical impedance Z. The reactance X can, for example, be purely capacitive and is then X = 1/(2$\pi$*f*C) with the frequency f of the electric voltage and with the electrical capacitance C. The reactance X can also, for example, be purely inductive and is then X = +2$\pi$*f*L with the frequency f and the inductance L.

The representation of the electrical impedance Z by polar coordinates

$$Z = Mod(Z) * exp(i*\phi) \qquad\qquad (2)$$

includes the modulus Mod(Z) and the phase angle $\phi$ of said electrical impedance Z.

Further, the electrical impedance Z can be represented by the cosine and sine functions of the phase angle $\phi$ :

$$Z = Mod(Z) * cos \phi + i * Mod(Z) * sin \phi. \qquad\qquad (3)$$

$$as\ exp(i*\phi) = cos \phi + i * sin \phi.$$

In particular the values of the cosine and sine functions of the phase angle $\phi$ are also parameters, which correlate with the type and properties of the food stuff. The determining or calculation of the cosine and/or sine of the phase angle $\phi$ from the electrical impedance Z provides further characteristic parameters of the food stuff.

Beside the cosine and sine functions, also the tangent and cotangent functions of the phase angle $\phi$ can be calculated from the electrical impedance Z. The trigonometric functions of the phase angle $\phi$ are suitable to provide other correlations with the properties of the food stuff as the phase angle $\phi$ itself, since each value of the trigonometric function corresponds with two values of the phase angle $\phi$.

Therefore, whenever in this application it is referred to an impedance Z to be detected or determined this implies that also one of the aforementioned components R, X, C, L, Mod(Z), $\phi$, sin $\phi$, cos $\phi$, etc. of the impedance Z alone or any combination thereof can be detected or determined as well.

According to the invention, correlations are used between measured values for or being dependent on the impedance Z itself or at least one of the ohmic resistance R, the reactance X, the modulus Mod(Z) and the phase angle $\phi$ or trigonometric functions thereof of the impedance Z for n=1 one single frequency or preferably n > 1 different frequencies f on the one hand and the types and/or properties of the food stuff on the other hand.

The values of the impedance Z or a component thereof at the n frequencies can be compared or correlated in different ways to determine the type or a property of the food stuff 20.

In a preferred embodiment two values (n = 2) at two different frequencies f1 and f2 are used, e.g. Z1 = Z(f1) or $\phi1 = \phi(f1)$ and Z2 = Z(f2) or $\phi2 = \phi(f2)$.

Alternatively, more than just two frequencies (n > 2), even a high number n of measuring frequencies of several hundred or even thousand can be used if a very detailed analysis with a high resolution is desired, for instance a detailed curve discussion or comparison of curve parameters of curves in a certain frequency spectrum.

Also it is possible to measure the impedance or component thereof several times in a given time interval, for instance every 30 seconds in the first 5 minutes of the cooking process, to average the measurements and decrease measuring inaccuracies.

Now, in order to determine a type or property, such as freshness, of the food stuff 20, a ratio of two values at the two different frequencies f1 and f2, e.g. Z1/Z2 or $\phi1/ \phi2$, can be used or a difference of two values at two different frequencies f1 and f2, e.g. Z1 - Z2 or $\phi1 - \phi 2$, or a ratio of a difference of two values at two different frequencies f1 and f2 and the sum of these two values at two different frequencies, e.g. (Z1 - Z2)/(Z1 + Z2) or $(\phi1 - \phi2)/(\phi1 + \phi 2)$.

Also another function apart from the described ratios or differences of the n values obtained for the n frequencies for n > 1 can be used to calculate parameters representing information on the food stuff from the detected values of the impedance or components thereof.

But it also possible to compare the two (or more) values of the impedance Z or its component at the corresponding frequencies with the same number of previously determined and stored reference values or reference data for different food types at the same frequency or frequencies and to decide where there is the highest degree of coincidence with the reference data of one of the stored food types by some kind of mathematical norm, for instance Euklidian norm, or metric (distance function).

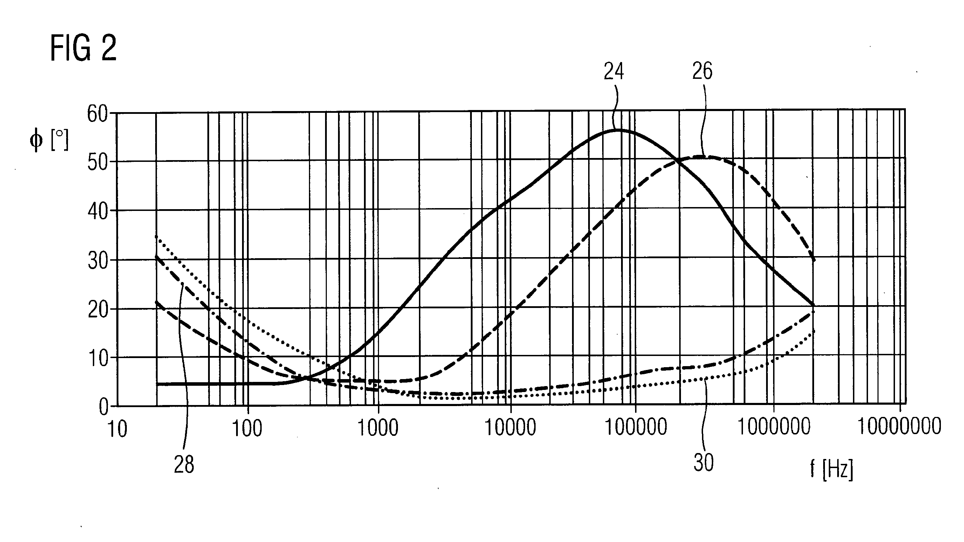

According to the invention it could be shown that different types of food or food stuff 20 have different and in each case characteristic impedances Z or components thereof at the same frequency or frequencies. Examples are shown in FIG 2.

FIG 2 illustrates in a schematic diagram typical functional graphs or curves of the value pairs (f, $\phi(f)$) of the detected or calculated phase angle $\phi$ of the impedance Z as a function of the frequency f in a given frequency spectrum for several types of food according to a preferred embodiment of the invention. These curves or graphs or sets of values are usually empirically determined by calibration or reference measurements using the food probe according to FIG 1 for probing different types of food stuff 20 and storing the data as reference data for the cooking process in the cooking oven or appliance.

The four curves 24, 26, 28 and 30 in FIG 2 show the phase angle $\phi$ over the same frequency spectrum for different types of food stuff 20. A first curve designated by 24 corresponds to potatoes as food stuff 20, a second curve 26 refers to cauliflower, a third curve 28 was determined for pork and a fourth curve 30 for the breast of a turkey hen. The frequency spectrum of the frequency f in FIG 2 extends from about 10 Hz to about 1 MHz.

FIG 2 clarifies that different types of food have their own characteristic phase angles $\phi$ as function of the frequency f and their respective curves 24, 26, 28 and 30 differ significantly in absolute values as well as shape of the curves. The vegetable curves 24 for potatoes and 26 for cauliflower both have a well defined maximum or peak but at different frequency as well as different value of the phase angle. The meat curves 28 and 30 both have a minimum in the middle and less variation in phase angle than the vegetable curves 24 and 26 and differ from each other in the absolute values of the phase angle at same frequencies (except for one single frequency where the two curves 28 and 30 intersect).

Therefore, by taking at least two values $\phi1 = \phi(f1)$ and $\phi2 = \phi(f2)$ of the phase angle $\phi$ at two different frequencies in each curve shown in FIG 2 the curve and thus the corresponding food stuff 20 can be unambigously determined or identified.

The phase angle φ in a specific frequency spectrum containing at least two frequency values can be detected for an actual food stuff 20 to be cooked, in particular by the food probe 10 and its associated evaluation and/or control unit, and then compared with a data base where calibration or reference curves, like the curves 24, 26, 28, 30, or look-up tables or the like are stored. Thus, the type of food can be automatically recognized. This applies to or is possible at different stages of the cooking process or different temperatures of the various food stuff 20.

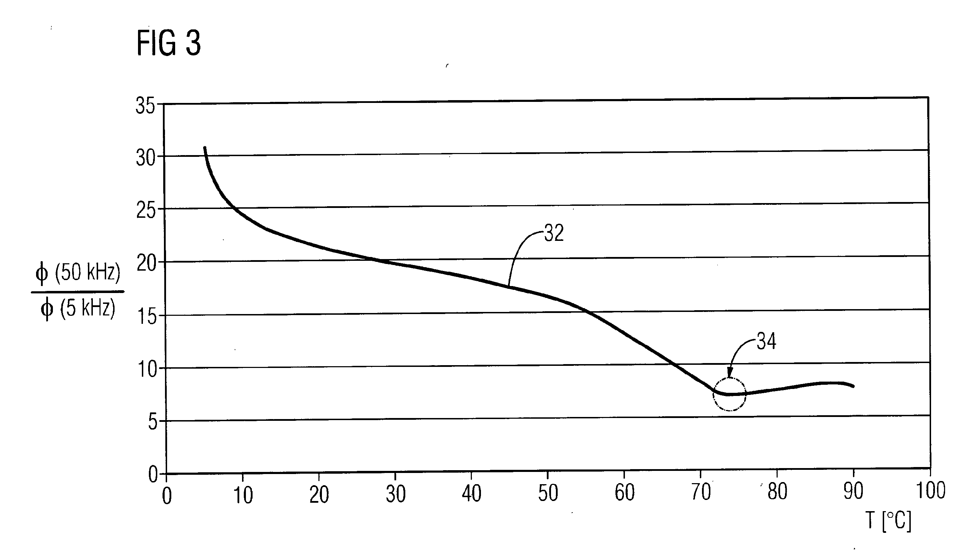

FIG 3 illustrates a schematic diagram of a ratio of two phase angles φ at different frequencies as a function of the temperature according to the preferred embodiment of the invention. The curve 32 relates to the ratio of the phase angles φ at the frequencies of 50 kHz and 5 kHz and relates to meat.

The interval of the temperature in FIG 3 extends from about 10°C to about 90 °C. At a point 34 the curve 32 has a minimum. At this point 34 basically all proteins of the food stuff are denatured, i.e. the meat is fully cooked. The point 34 corresponds with a temperature between 70°C and 80 °C.

This example in FIG 3 shows that an electrical parameter or variable derived from the impedance Z such as in this case the ratio of the phase angles at two different frequencies can also be used to determine the temperature or to control the cooking process in time in particular by determining the degree or progress of cooking without actually having to measure the temperature directly.

In general it can be said that the impedance Z of the food stuff depends on the degree of destruction in the cells of the food stuff by the cooking process.

So also by these measurements at further stages of the cooking process it is possible to determine or further specify the type of food as different types of food as well as also food of different age show different degradation or denaturation during the cooking process.

After having determined the type of the food stuff 20 which is probed by the food probe 10 or as an alternative or in addition to such determination of the food type according to the invention it is also possible, in a further embodiment according to the invention, to determine also properties or characteristics of the food stuff such as its age by using the impedance or at least one component thereof. The age of the food stuff 20, in particular meat such as poultry or pork or veal or fish etc., can then be used by the control unit of the cooking appliance or oven in order to control the cooking process accordingly, in particular to control the cooking time and/or cooking temperature. Aged food stuff, in particular meat or other food obtained from animals, will usually contain more microorganisms, in particular bacteria, and will thus need more time or higher temperature to sterilize and kill the microorganisms sufficiently. An example of such determination of the age of food stuff will be given in the following.

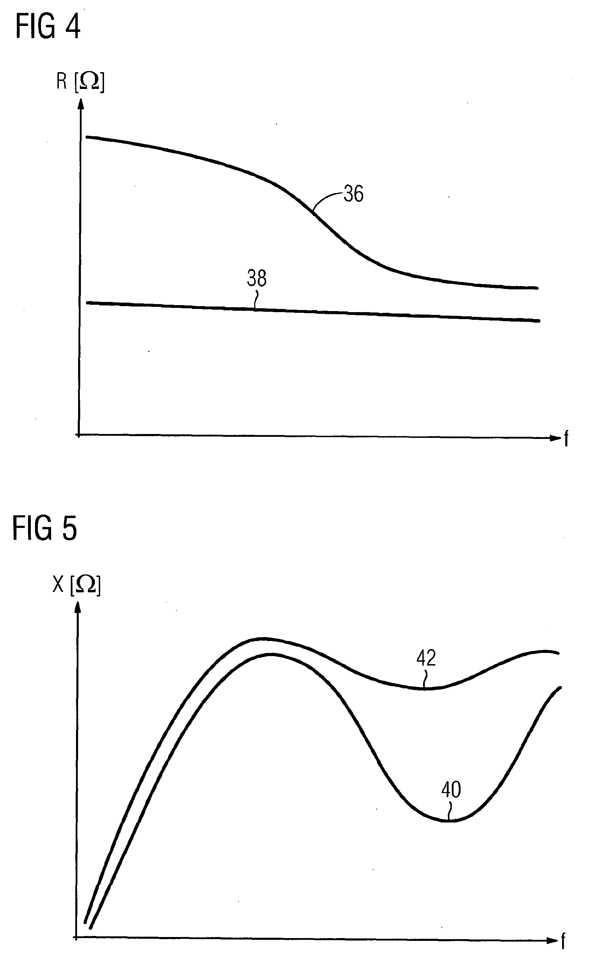

FIG 4 illustrates a schematic diagram of the ohmic resistance R measured for poultry of different age as a function of the frequency f according to the preferred embodiment of the present invention. The ohmic resistance R is the real part of the complex electrical impedance Z. The electrical impedance Z is detected and the ohmic resistance R is then calculated from said detected electrical impedance Z or the ohmic resistance R can also be detected or measured directly.

A typical curve or functional graph of the pairs (f, R(f)) of the value of the detected or calculated ohmic resistance R as a function of the frequency f of fresh poultry is designated by 36 and a corresponding graph or curve of aged poultry by 38. At low frequencies f, the ohmic resistance R of the fresh poultry according to curve 36 is much higher than the ohmic resistance R of aged poultry according to curve 38. The ohmic resistance R of the fresh poultry according to curve 36 decreases clearly with the increasing frequency f and then approaches a roughly constant value again. The ohmic resistance R of the aged poultry according to curve 38 however decreases much less or only marginally with the increasing frequency f and remains almost constant. At high frequencies f, the ohmic resistance R of the fresh poultry according to curve 36 is still a bit higher than the ohmic resistance R of aged poultry according to curve 38.

Thus, the dependency or correlation of the ohmic resistance R of the electrical impedance Z over the same frequency spectrum or interval shown in FIG 4 differs significantly for the fresh poultry according to curve 36 and the aged poultry according to curve 38 or, in other words the functions R(f) for fresh poultry and less fresh or aged poultry are different in absolute values as well as in the first derivative $dR/df$ and the second derivative $d^2R/d^2f$ for instance. Also the curve 36 for fresh poultry may have a turning point or inflection point in contrast to curve 38 for aged poultry.

FIG 5 shows in a schematic diagram the reactance X as a function of the frequency f for poultry of different age according to the preferred embodiment of the present invention. The reactance X is the imaginary part of the complex electrical impedance Z. The electrical impedance Z is detected and the reactance X is then calculated from said detected electrical impedance Z or, again, the reactance X is measured directly.

The curve designated by 40 shows the reactance X of fresh poultry and the curve 42 the reactance X of aged poultry each as a function of the frequency f. At low frequencies f, the reactance X(f) of the fresh poultry according to curve 40 as well as the reactance X of the aged poultry according to curve 42 are small and rise with the increasing frequency f. The reactance X of the fresh poultry in curve 40 has two maxima and a minimum between said maxima. The reactance X of the aged poultry in curve 42 has also two maxima and a minimum at the same frequency ranges. A first maximum is in the central frequency range, and a second maximum is at high frequencies f.

The minima of the reactance X are the most significant difference between the fresh poultry according to curve 40 and the aged poultry according to curve 42. The reactance X of the aged poultry according to curve 42 at the minimum is only marginally smaller than the reactance X of the aged poultry according to curve 42 at the maxima. However, the reactance X of the fresh poultry according to curve 40 at the minimum is substantially lower than the reactance X of the fresh poultry according to curve 40 at the maxima. Further, the reactance X of the fresh poultry according to curve 40 at the minimum is clearly smaller than the reactance X of the aged poultry according to curve 42 at the minimum.

Thus, the curves for the reactance X of the electrical impedance Z over the same frequency spectrum differ significantly for the fresh poultry (curve 40) and the aged poultry (curve 42). in other words the functions X(f) for fresh poultry and aged poultry are different in absolute values as well as in the first derivative dR/df and the second derivative $d^2R/d^2f$ at same frequencies for instance.

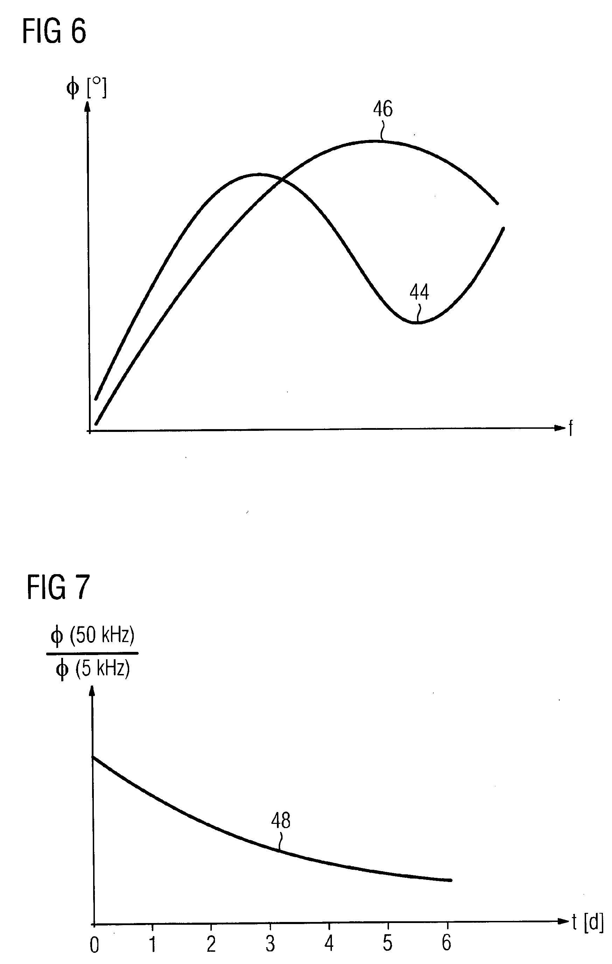

FIG 6 illustrates a schematic diagram of the phase angle $\phi$ as a function of the frequency f for poultry of different age according to the preferred embodiment of the present invention. The phase angle $\phi$ is the phase part of the complex electrical impedance Z. The electrical impedance Z is detected and the phase angle $\phi$ is then calculated from said detected electrical impedance Z or the phase angle $\phi$ is detected directly.

As can be seen in FIG 6, at low frequencies f, the phase angle $\phi$ of the fresh poultry depicted in curve 44 is small and rises with the increasing frequency f. Two maxima occur in the central frequency range and at high frequencies f in curve 44 for fresh poultry, and there is a clear minimum between the both maxima. In contrast, the phase angle $\phi$ of the aged poultry in curve 46 has only one maximum and no minima. Thus, the phase angle $\phi$ of the electrical impedance Z differs significantly for the fresh poultry according to curve 44 and the aged poultry according to curve 46 in the same frequency spectrum.

In FIG 7 the ratio of two phase angles $\phi$ at different frequencies f is depicted as a function of the age t in days (d) of the food stuff according to the preferred embodiment of the present invention. In this example, the frequencies are 50 kHz and 5 kHz. The curve 48 relates to poultry. FIG 7 shows that the ratio of the two phase angles $\phi$ decreases with an increasing age of the poultry. Thus, the user can check the quality of the poultry or food stuff 20 in general at the beginning of the cooking process by using this ratio for instance.

As was shown by means of FIG 4 to 7 different components of the impedance Z of the food stuff 20 or parameters derived thereof can be used to determine the age or freshness of the food stuff, preferably before or at the beginning of the cooking process.

Accordingly, reference data for the characteristic functional relationship R(f) in FIG 4 or X(f) in FIG 5 or $\phi$(f) in FIG 6 or $\phi$(f1)/$\phi$(f2) in FIG 7 for the various food stuff 20 of different age provided as cooking goods, in particular meat or animals to be cooked such as poultry or pork or veal or fish etc., can be empirically obtained by calibration measurements or calculated by interpolation or extrapolation. The reference data is stored in a storage or data base and used as reference data for comparison with actual data determined at the beginning or during a cooking process in order to determine the age of the poultry, meat or food stuff 20 in general which is going to be or is being cooked.

Instead of a ratio of the phase angles at two frequencies also a ratio of the capacitances at two frequencies could be used or of any other component of the impedance Z.

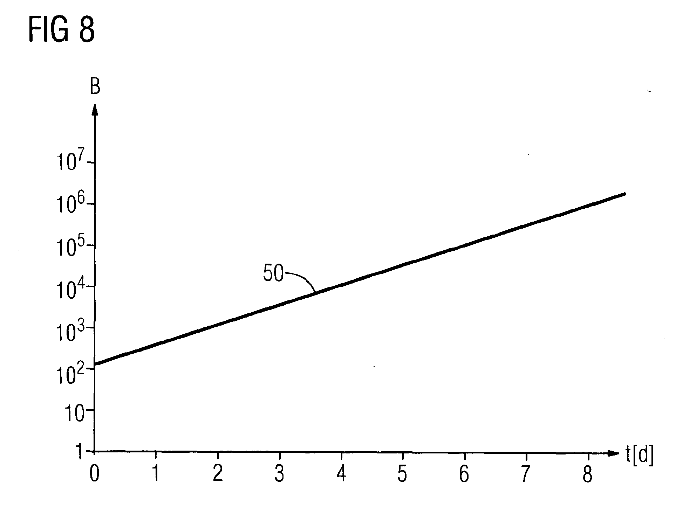

FIG 8 illustrates a schematic diagram of an amount B of bacteria as a function of the age t of the food stuff according to the preferred embodiment of the present invention. In this example, the shown bacterium is listeria monocytogenes. The food stuff is poultry. The temperature T is 5°C. It can be seen that the amount B of bacteria increases tenfold within two days. After eight days the amount B of bacteria increases by the factor of 10.000.

So from FIG 7 and the correlation between the ratio of phase angles $\phi$ at the frequencies f of 50 kHz and 5 kHz and the amount B of bacteria in FIG 8 at the same age t the user can determine not only the age of the poultry or food stuff in general but also estimate the amount B of bacteria.

The examples shown by the drawings have clarified that several coordinates or components of the electrical impedance Z provide substantial information about the food stuff.

Further parameters like capacitance and specific dielectric constants can be calculated from the detected quantities.

Additionally, the first and/or second derivative of the detected and/or calculated quantities or parameters can be determined.

In order to obtain more information the temperature of the food stuff 20 can be detected additionally.

A teach-in function can be implemented. Such a teach-in function allows the user a possibility to train the cooking oven for recognizing individual recipes or food stuff, which are not yet in the data base.

The present invention can also be embedded in a computer program product, which comprises all the features enabling the implementation of the method described herein. Further, when loaded in a computer system, said computer program product is able to carry out these methods.

According to the preferred embodiment of the present invention the electrical parameters are coordinates of the electrical impedance in the complex plane. The complex plane can be represented by a number of coordinate systems. The electrical parameters are formed by the according coordinates. The present invention bases on the cognition, that there is a correlation between the coordinates of the electrical impedance in the complex plane and the properties of the food stuff.

For example, the electrical parameters are Cartesian coordinates of the electrical impedance in the complex plane. The Cartesian coordinates of the electrical impedance in the complex plane are the ohmic resistance and the reactance. In general, the ohmic resistance and the reactance of the food stuff have different relationships to the quality of the food stuff. Thus, the ohmic resistance and the reactance contain more information of the food stuff as one of these parameters.

According to a further example, the electrical parameters are polar coordinates of the electrical impedance in the complex plane. The polar coordinates of the electrical impedance in the complex plane are the modulus and the phase angle of the electrical impedance.

Further, the electrical parameters may be functions of at least one coordinate of the electrical impedance in the complex plane. Such functions of the coordinates of the electrical impedance are preferred, which show a significant dependence of the properties of the food stuff. For example, the trigonometric functions of the phase angle can be used.

According to another embodiment of the present invention, a ratio of phase angles of the electrical impedance at two different frequencies is calculated as a function of the temperature of the food stuff. This ratio of phase angles depends on the temperature of the food stuff, so that the temperature inside the food stuff can be determined.

Further, the first and/or second derivatives of the detected and/or calculated parameters may be determined.

In particular, the data base comprises frequency spectra of the electrical impedance and/or of the calculated electrical parameters. For example, the data base may comprise the frequency spectra of the phase angle of the electrical impedance for a plurality of types of food stuff.

According to a special embodiment of the present invention, the data base can be supplemented by the user. Thus, the user can adapt the data base to individual recipes. Such a teach-in function allows the user a possibility to train the cooking oven for recognizing individual recipes, which are not yet in the data base.

Additionally, further electrical parameters of the food stuff may be calculated from the electrical impedance. For example, the capacity of the food stuff is calculated from the electrical impedance.

**List of reference numerals**

| | |
|---|---|
| 10 | food probe |
| 12 | rod |
| 14 | first electrode |
| 16 | second electrode |
| 18 | spike |
| 20 | food stuff |
| 22 | electric voltage |
| 24 | frequency spectrum of phase angle for potatoes |
| 26 | frequency spectrum of phase angle for cauliflowers |
| 28 | frequency spectrum of phase angle for pork |
| 30 | frequency spectrum of phase angle for turkey |
| 32 | ratio of phase angle as function of temperature |
| 34 | point where all proteins are denaturated |
| 36 | curve of ohmic resistance of fresh poultry |
| 38 | curve of ohmic resistance of aged poultry |
| 40 | curve of reactance of fresh poultry |
| 42 | curve of reactance of aged poultry |
| 44 | curve of ohmic resistance of fresh poultry |
| 46 | curve of ohmic resistance of aged poultry |
| 48 | curve of ratio of phase angle as function of time |
| 50 | amount of bacteria |
| Z | electrical impedance |
| R | ohmic resistance |
| X | reactance |
| Mod(Z) | modulus of the electrical impedance Z |
| $\phi$ | phase angle of the electrical impedance Z |
| f | frequency |
| T | temperature |
| t | time |

B        amount of bacteria

**Claims**

1. A method for obtaining information on food stuff (20) in or for a cooking process comprising the steps of:

   a) detecting an electrical impedance (Z) or at least one component thereof in a, preferably inner, region of the food stuff (20) by applying at least one electric voltage (22) to said region of the food stuff (20) and measuring the impedance (Z) or the at least one component thereof in that region of the food stuff, using at least one food probe (10) which is inserted into the food stuff (20) and comprises at least two electrodes (14, 16) for applying the electric voltage (22) and measuring the impedance (Z) or the at least one component thereof, wherein the at least one electrical component of the electrical impedance (Z) is chosen from the group comprising the ohmic resistance (R), the reactance (X), the capacity, the inductivity, the modulus and the phase angle ($\phi$),
   b) wherein the electrical impedance (Z) or the at least one component thereof is detected at two or more frequencies (f) of the electric voltage (22) applied,
   c) deriving from said detected impedance or component thereof information about at least one characteristic property of the food stuff,
   d) at least one characteristic property of the food stuff (20) being the type of food
   e) wherein the step b) of deriving information about the characteristic property of the food stuff from said detected impedance or component thereof is performed by comparison with stored pre-determined reference data,
   f) using in said step of deriving of information on the characteristic property of the food stuff a ratio of two detected values of the impedance or component thereof at two different frequencies or a difference or sum of two detected values of the impedance or component thereof at two different frequencies or a ratio of a difference and the sum of two detected values of the impedance or component thereof at two different frequencies,
   g) providing said information about the characteristic property of the food stuff to a user interface and/or to an information output unit and/or to a control unit for controlling the cooking process.

2. The method according to claim 1, comprising the following step:

   deriving from said detected impedance or component thereof information about at least one further characteristic property of the food stuff, being at least one of the age or freshness of the food stuff (20), the content of fat of the food stuff (20), and whether the food stuff (20) was frozen before or not.

3. The method according to claim 2, comprising at least one or an arbitrary combination of the following steps or features:

   a) deriving the information about the food type of the food stuff, including in particular different types of meat and/or fish and/or vegetables, from said detected impedance or component thereof before said step of deriving information about the age or freshness of the food stuff and/or based on the same detected impedance or component values as said step of deriving information about the age or freshness of the food stuff,
   b) using said information about the characteristic property of the food stuff for adapting the cooking time and/or cooking temperature of the cooking process dependent on this information, wherein in particular for less fresh or aged food stuff and/or for food stuff already frozen before the cooking time is selected to be longer and/or the cooking temperature is selected to be higher or to be at a high level for a longer time.

4. The method according to one of claims 1 to 3, wherein the reference data can be supplemented by the user.

5. The method according to one of claims 1 to 4, comprising at least one or an arbitrary combination of the following steps or features:

   a) said step of detecting the electrical impedance (Z) or the at least one component thereof of the food stuff for a subsequent deriving of information on the characteristic property of the food stuff takes place before or at the beginning of the cooking process and/or in a predefined temperature range, preferably low, temperature e.g. room temperature,
   b) in said step of detecting the electrical impedance (Z) or the at least one component thereof of the food stuff several values are measured in a pre-given time interval, e.g. several minutes, at pre-determined instants of time, e.g. every 10 to 60 seconds.

**6.** The method according to one of claims 1 to 5,

a) wherein for n > 1 frequencies m measuring steps are performed with $0 < m \leq n$ in each of which a voltage is applied wherein each of the n frequencies is contained in at least one of the applied voltages and/or

b) wherein one of the two different frequencies (f) is chosen to be 50 kHz and another one of the two different frequencies (f) is chosen to be 5 kHz.

**7.** The method according to claim 6, comprising at least one or an arbitrary combination of the following steps or features:

a) comparing the detected values of the impedance or component thereof at the one, two or more frequencies with the same number of stored reference values for characteristic properties of food stuff previously determined at the same frequencies and determining the characteristic property of the food stuff by determining the highest degree of coincidence with the reference values, e.g. by some mathematical norm, for instance Euklidian norm, or metric,

b) using in said step of deriving of information on the characteristic property of the food stuff a function or calculation algorithm of the detected values of the impedance or component thereof at the one, two or more frequencies.

**8.** The method according to one of claims 1 to 7, comprising at least one or an arbitrary combination of the following steps or features:

a) using in said step of deriving information about the characteristic property, in particular the age or freshness and/or the type, of the food stuff the ohmic resistance (R) as the component of the impedance,

b) the ohmic resistance (R) over the same frequency spectrum for different characteristic properties, in particular for different food types as well as for different food age, differs in absolute values as well as in the first derivative and the second derivative,

c) at lower frequencies, the ohmic resistance (R) of fresh food stuff, in particular meat such as poultry, is higher than that of aged food stuff and/or wherein the ohmic resistance (R) of fresh food stuff decreases more steeply with increasing frequency than that of aged food stuff and/or wherein, at high frequencies (f), the ohmic resistance (R) of the fresh food stuff is higher than that of aged food stuff and/or wherein the curve of the ohmic resistance for fresh food stuff has a turning point in contrast to aged food stuff.

**9.** The method according to one of claims 1 to 8, comprising at least one or an arbitrary combination of the following steps or features:

a) using in said step of deriving information about the characteristic property of the food stuff the reactance (X) as the component of the impedance,

b) the reactance over the same frequency spectrum for different characteristic properties, in particular for different food types as well as for different food age, differs in absolute values as well as in the first derivative and the second derivative,

c) the reactance of fresh food stuff, in particular meat such as poultry, as well as of aged food stuff has a minimum in a given frequency spectrum and the reactance (X) of the fresh food stuff at the minimum is smaller than the reactance (X) of the aged food stuff at the minimum.

**10.** The method according to one of claims 1 to 9, comprising at least one or an arbitrary combination of the following steps or features:

a) using in said step of deriving information about the characteristic property of the food stuff the phase angle ($\phi$) as the component of the impedance,

b) the phase angle ($\phi$) over the same frequency spectrum for different characteristic properties, in particular for different food types as well as for different food age, differs in absolute values as well as in the first derivative and the second derivative,

c) the phase angle ($\phi$) of fresh food stuff, in particular meat such as poultry, has a minimum in a given frequency spectrum whereas the phase angle ($\phi$) of aged food stuff has no minimum in this given frequency spectrum,

d) the ratio of phases angles ($\phi$) at two different frequencies (f) decreases with increasing age of the food stuff and is in particular used to check the quality of the food stuff before or at the beginning of the cooking process.

e) the phase angle has for vegetable a well defined maximum in a given frequency spectrum but at a different frequency for different vegetables as well as a different absolute value,

f) the phase angle has for different meat different absolute values at same frequencies.

11. The method according to one of claims 1 to 10, comprising at least one or an arbitrary combination of the following steps or features:

a) deriving from said detected impedance or component thereof information about characteristic property of the food stuff by calculating one or more electrical parameters (R; X; $\phi$) of the food stuff (20) from the electrical impedance (Z) or component thereof and comparing said electrical parameters (R; X; $\phi$) with a data base,

b) detecting, in another step during the cooking process and/or when the food stuff has been subjected to cooking already, the electrical impedance (Z) or the at least one component thereof of the food stuff again for determining the cooking progress and/or cooking temperature in the region of the food stuff,

c) wherein in particular a ratio of phase angles ($\phi$) at two different frequencies (f) is calculated as a function of the temperature (T) of the food stuff (20),

d) deriving further to or as the information about the age or freshness of the food stuff an information on the estimated amount or concentration of bacteria in the food stuff.

12. A food probe (10) for inserting into a food stuff (20), being provided for use in the method according to any one of the claims 1 to 11 and comprising:

- an elongated rod (12) made of a non-conductive material,
- a front portion of the elongated rod (12), which front portion is provided for invading into the food stuff (20),
- a first electrode (14) made of a conductive material and arranged at the front portion of the rod (12),
- a second electrode (16) made of a conductive material and arranged at the front portion of the rod (12) in a predetermined distance from the first electrode (14), and
- a spike (18) arranged at a front end of the rod (12), wherein
- the first electrode (14) and the second electrode (16) are arranged serially along the longitudinal axis of the rod (12),
- the food probe (10) is connected or connectable to a voltage supply and a control circuit of a cooking oven or cooking appliance, and
- a voltage can be applied between the first electrode (14) and the second electrode (16), so that an electrical voltage (22) is generated between and in the environment of the first electrode (14) and the second electrode (16).

**Patentansprüche**

1. Verfahren zur Informationsgewinnung über Lebensmittel (20) in einem oder für einen Kochprozess, folgende Schritte umfassend:

a) Feststellen einer elektrischen Impedanz (Z) oder wenigstens einer Komponente davon in einem vorzugsweise inneren Bereich des Lebensmittels (20) durch Anlegen wenigstens einer elektrischen Spannung (22) an den Bereich des Lebensmittels (20) und Messen der Impedanz (Z) oder der wenigstens einen Komponente davon in diesem Bereich des Lebensmittels unter Anwendung wenigstens einer Lebensmittelsonde (10), die in das Lebensmittel (20) eingeführt wird und wenigstens zwei Elektroden (14, 16) zum Anlegen der elektrischen Spannung (22) und Messen der Impedanz (Z) oder der wenigstens einen Komponente davon umfasst, wobei die wenigstens eine elektrische Komponente der elektrischen Impedanz (Z) ausgewählt ist aus der Gruppe umfassend den Ohmschen Widerstand (R), die Reaktanz (X), die Kapazität, die Induktivität, den Modul und den Phasenwinkel ($\phi$),

b) wobei die elektrische Impedanz (Z) oder die wenigstens eine Komponente davon bei zwei oder mehr Frequenzen (f) der angelegten elektrischen Spannung (22) ermittelt wird,

c) Ableiten von Informationen über wenigstens eine charakteristische Eigenschaft des Lebensmittels von der festgestellten Impedanz oder der Komponente davon,

d) wobei wenigstens eine charakteristische Eigenschaft des Lebensmittels (20) die Art des Lebensmittels ist,

e) wobei der Schritt b) des Ableitens von Informationen über die charakteristische Eigenschaft des Lebensmittels von der festgestellten Impedanz oder der Komponente davon über einen Vergleich mit gespeicherten, vorher ermittelten Referenzdaten durchgeführt wird,

f) wobei in dem Schritt des Ableitens von Informationen über die charakteristische Eigenschaft des Lebensmittels ein Verhältnis zwischen zwei festgestellten Werten der Impedanz oder der Komponente davon bei zwei unterschiedlichen Frequenzen oder eine Differenz oder Summe zweier festgestellter Werte der Impedanz oder der Komponente davon bei zwei unterschiedlichen Frequenzen oder ein Verhältnis einer Differenz und der Summe zweier festgestellter Werte der Impedanz oder einer Komponente davon bei zwei unterschiedlichen Frequenzen zur Anwendung kommt,

g) Bereitstellen der Informationen über die charakteristische Eigenschaft des Lebensmittels an eine Benutzeroberfläche und/oder an eine Informationsausgabeeinheit und/oder an eine Steuerungseinheit zur Steuerung des Kochprozesses.

2. Verfahren gemäß Anspruch 1, das folgenden Schritt umfasst:

Ableiten von Informationen über wenigstens eine weitere charakteristische Eigenschaft des Lebensmittels von der festgestellten Impedanz oder der Komponente davon, wobei es sich wenigstens um eine der Eigenschaften des Alters oder der Frische des Lebensmittels (20), des Fettgehalts des Lebensmittels (20) und ob das Lebensmittel (20) zuvor eingefroren war oder nicht handelt.

3. Verfahren gemäß Anspruch 2, das wenigstens einen oder eine beliebige Kombination der nachfolgenden Schritte oder Merkmale umfasst:

a) Ableiten der Informationen über die Lebensmittelart des Lebensmittels, insbesondere einschließlich verschiedener Arten von Fleisch und/oder Fisch und/oder Gemüse, von der festgestellten Impedanz oder einer Komponente davon vor dem Schritt des Ableitens von Informationen über das Alter oder die Frische des Lebensmittels und/oder basierend auf denselben Impedanz- oder Komponentenwerten wie der Schritt des Ableitens von Informationen über das Alter oder die Frische des Lebensmittels,

b) Verwendung der Informationen über die charakteristische Eigenschaft des Lebensmittels zur Einstellung der Kochzeit und/oder der Kochtemperatur des Kochprozesses in Abhängigkeit von diesen Informationen, wobei insbesondere für weniger frische oder ältere Lebensmittel und/oder für zuvor bereits eingefroren gewesene Lebensmittel eine längere Kochzeit und/oder eine höhere Kochtemperatur oder eine über längere Zeit auf hohem Niveau liegende Kochtemperatur ausgewählt wird.

4. Verfahren gemäß einem der Ansprüche 1 bis 3, wobei die Referenzdaten vom Benutzer eingebracht werden können.

5. Verfahren gemäß einem der Ansprüche 1 bis 4, das wenigstens einen oder eine beliebige Kombination der folgenden Schritte oder Merkmale umfasst:

a) den Schritt der Feststellung der elektrischen Impedanz (Z) des Lebensmittels oder der wenigstens einen Komponente davon für eine nachfolgende Ableitung von Informationen über die charakteristische Eigenschaft des Lebensmittels findet vor oder zu Beginn des Kochprozesses und/oder in einem vorgegebenen Temperaturbereich statt, vorzugsweise bei niedriger Temperatur, zum Beispiel bei Raumtemperatur,

b) bei dem Schritt der Feststellung der elektrischen Impedanz (Z) des Lebensmittels oder der wenigstens einen Komponente davon werden mehrere Werte in einem vorgegebenen Zeitintervall, zum Beispiel in einigen Minuten, zu vorgegebenen Zeitpunkten, zum Beispiel alle 10 bis 60 Sekunden gemessen.

6. Verfahren gemäß einem der Ansprüche 1 bis 5,

a) wobei für n > 1 Frequenzen m Messschritte durchgeführt werden, wofür $0 < m \leq n$ gilt, in deren jedem eine Spannung angelegt wird, wobei jede der n Frequenzen in wenigstens einer der angelegten Spannungen enthalten ist, und/oder

b) wobei eine der zwei unterschiedlichen Frequenzen (f) mit 50 kHz und eine andere der zwei unterschiedlichen Frequenzen (f) mit 5 kHz ausgewählt wird.

7. Verfahren gemäß Anspruch 6, das wenigstens einen oder eine beliebige Kombination der folgenden Schritte oder Merkmale umfasst:

a) Vergleichen der festgestellten Impedanzwerte oder der Komponente davon bei der einen, den zwei oder den mehreren Frequenzen mit derselben Anzahl gespeicherter Referenzwerte für charakteristische Eigenschaften von Lebensmitteln, die zuvor bei denselben Frequenzen festgestellt wurden, und Bestimmen der charakteristischen Eigenschaft des Lebensmittels durch Bestimmen des höchsten Maßes an Koinzidenz mit den Referenzwerten, zum Beispiel anhand einer mathematischen Norm, beispielsweise der euklidischen oder metrischen Norm,

b) Verwendung einer Funktion oder eines Rechenalgorithmus der festgestellten Werte der Impedanz oder der Komponente davon bei der einen, den zwei oder mehr Frequenzen in dem Schritt der Ableitung von Informationen über die charakteristische Eigenschaft des Lebensmittels.

8. Verfahren gemäß einem der Ansprüche 1 bis 7, das wenigstens einen oder eine beliebige Kombination der folgenden Schritte oder Merkmale umfasst:

a) Verwendung des Ohmschen Widerstands (R) als Komponente der Impedanz in dem Schritt der Ableitung von Informationen über die charakteristische Eigenschaft, insbesondere das Alter oder die Frische und/oder die Art des Lebensmittels,
b) der Ohmsche Widerstand (R) im selben Frequenzspektrum unterschiedlicher charakteristischer Eigenschaften, insbesondere für unterschiedliche Lebensmittelarten sowie für unterschiedliches Alter der Lebensmittel, unterscheidet sich in absoluten Werten ebenso wie in der ersten Ableitung und in der zweiten Ableitung,
c) bei tieferen Frequenzen ist der Ohmsche Widerstand (R) frischer Lebensmittel, insbesondere von Fleisch wie etwa Geflügel, höher als jener älterer Lebensmittel und/oder wobei der Ohmsche Widerstand (R) frischer Lebensmittel bei zunehmender Frequenz schneller abnimmt als jener von älteren Lebensmitteln und/oder wobei der Ohmsche Widerstand (R) der frischen Lebensmittel bei hohen Frequenzen (f) höher ist als jener älterer Lebensmittel und/oder wobei die Kurve des Ohmschen Widerstands für frische Lebensmittel im Gegensatz zu älteren Lebensmitteln einen Wendepunkt aufweist.

9. Verfahren gemäß einem der Ansprüche 1 bis 8, das wenigstens einen oder eine beliebige Kombination der folgenden Schritte oder Merkmale umfasst:

a) in dem Schritt der Ableitung von Informationen über die charakteristische Eigenschaft des Lebensmittels die Verwendung der Reaktanz (X) als Komponente der Impedanz,
b) im selben Frequenzspektrum unterschiedlicher charakteristischer Eigenschaften unterscheidet sich die Reaktanz, insbesondere für unterschiedliche Lebensmittelarten sowie für unterschiedliches Alter der Lebensmittel, in absoluten Werten ebenso wie in der ersten Ableitung und in der zweiten Ableitung,
c) die Reaktanz frischer Lebensmittel, insbesondere von Fleisch wie etwa Geflügel, hat ein Minimum in einem bestimmten Frequenzspektrum, und die Reaktanz (X) des frischen Lebensmittels ist bei dem Minimum kleiner als die Reaktanz (X) des älteren Lebensmittels bei dem Minimum.

10. Verfahren gemäß einem der Ansprüche 1 bis 9, das wenigstens einen oder eine beliebige Kombination der folgenden Schritte oder Merkmale umfasst:

a) in dem Schritt der Ableitung von Informationen über die charakteristische Eigenschaft des Lebensmittels die Verwendung des Phasenwinkels ($\phi$) als Komponente der Impedanz,
b) der Phasenwinkel ($\phi$) unterscheidet sich über dasselbe Frequenzspektrum für unterschiedliche charakteristische Eigenschaften, insbesondere für unterschiedliche Lebensmittelarten sowie für unterschiedliche Lebensmittelalter, in absoluten Werten sowie in der ersten Ableitung und der zweiten Ableitung,
c) der Phasenwinkel ($\phi$) von frischen Lebensmitteln, insbesondere von Fleisch wie etwa Geflügel, hat in einem bestimmten Frequenzspektrum ein Minimum, während der Phasenwinkel ($\phi$) von älteren Lebensmitteln in diesem gegebenen Frequenzspektrum kein Minimum aufweist,
d) das Verhältnis der Phasenwinkel ($\phi$) bei zwei unterschiedlichen Frequenzen (f) nimmt mit zunehmendem Alter der Lebensmittel ab und wird insbesondere dazu verwendet, die Qualität der Lebensmittel vor und zu Beginn des Kochprozesses zu prüfen,
e) der Phasenwinkel hat für Gemüse ein gut definiertes Maximum in einem gegebenen Frequenzspektrum, aber bei einer unterschiedlichen Frequenz für unterschiedliche Gemüse sowie bei einem unterschiedlichen absoluten Wert,
f) der Phasenwinkel hat für unterschiedliche Fleischsorten unterschiedliche absolute Werte bei denselben Frequenzen.

11. Verfahren gemäß einem der Ansprüche 1 bis 10, das wenigstens einen oder eine beliebige Kombination der folgenden Schritte oder Merkmale umfasst:

a) Ableitung von Informationen über charakteristische Eigenschaften des Lebensmittels von der festgestellten Impedanz oder der Komponente davon durch Berechnung eines oder mehrerer elektrischer Parameter (R; X; $\phi$) des Lebensmittels (20) von der elektrischen Impedanz (Z) oder der Komponente davon und Vergleichen der elektrischen Parameter (R; X; ϕ) mit einer Datenbank,

b) in einem anderen Schritt während des Kochprozesses und/oder nachdem das Lebensmittel dem Kochvorgang bereits ausgesetzt war das erneute Feststellen der elektrischen Impedanz (Z) des Lebensmittels oder der wenigstens einen Komponente davon, um den Kochfortschritt und/oder die Kochtemperatur im Bereich des Lebensmittels festzustellen,

c) wobei insbesondere ein Verhältnis von Phasenwinkeln (ϕ) bei zwei unterschiedlichen Frequenzen (f) als Funktion der Temperatur (T) des Lebensmittels (20) berechnet wird,

d) zusätzlich zu den Informationen oder als die Informationen über das Alter oder die Frische des Lebensmittels die Ableitung von Informationen über die geschätzte Menge oder Konzentration von Bakterien in dem Lebensmittel.

12. Lebensmittelsonde (10) zum Einführen in ein Lebensmittel (20), die zur Verwendung in dem Verfahren gemäß einem der Ansprüche 1-11 vorgesehen ist und Folgendes umfasst:

- einen langgestreckten Stab (12) aus einem nicht leitenden Material,
- einen vorderen Teil des langgestreckten Stabes (12), wobei der vordere Teil zum Eindringen in das Lebensmittel (20) vorgesehen ist,
- eine erste Elektrode (14) aus einem leitenden Material, die am vorderen Teil des Stabes (12) angeordnet ist,
- eine zweite Elektrode (16) aus einem leitenden Material, die an dem vorderen Teil des Stabes (12) in einem vorher festgelegten Abstand von der ersten Elektrode (14) angeordnet ist, und
- eine Spitze (18), die am vorderen Ende des Stabes (12) angeordnet ist, wobei
- die erste Elektrode (14) und die zweite Elektrode (16) seriell entlang der Längsachse des Stabes (12) angeordnet sind,
- die Lebensmittelsonde (10) an eine Spannungsquelle und einen Steuerungsschaltkreis eines Küchenofens oder eines Kochgeräts angeschlossen oder anschließbar ist, und
- zwischen der ersten Elektrode (14) und der zweiten Elektrode (16) eine Spannung angelegt werden kann, so dass zwischen und in der Umgebung der ersten Elektrode (14) und der zweiten Elektrode (16) eine elektrische Spannung (22) erzeugt wird.

## Revendications

1. Procédé pour obtenir des informations sur des aliments (20) dans ou pour un processus de cuisson, comprenant les étapes de :

a) détecter une impédance électrique (Z) ou au moins une composante de celle-ci dans une région, de préférence intérieure, de l'aliment (20) en appliquant au moins une tension électrique (22) à ladite région de l'aliment (20) et en mesurant l'impédance (Z) ou au moins une composante précitée de celle-ci dans cette région de l'aliment, en utilisant au moins une sonde d'aliment (10) qui est insérée dans l'aliment (20) et comprend au moins deux électrodes (14, 16) pour appliquer la tension électrique (22) et pour mesurer l'impédance (Z) ou au moins une composante précitée de celle-ci, où au moins une composante électrique précitée de l'impédance électrique (Z) est sélectionné dans le groupe comprenant la résistance ohmique (R), la réactance (X), la capacité, l'inductivité, le module et l'ange de phase (φ),

b) où l'impédance électrique (Z) ou au moins une composante précitée de celle-ci est détectée à deux ou plusieurs fréquences (f) de la tension électrique (22) appliquée,

c) dériver de ladite impédance détectée ou de la composante de celle-ci des informations se rapportant à au moins une propriété caractéristique de l'aliment,

d) au moins une propriété caractéristique de l'aliment (20) étant le type d'aliment,

e) où l'étape b) consistant à dériver des informations se rapportant à la propriété caractéristique de l'aliment à partir de ladite impédance détectée ou composante de celle-ci est exécutée en comparaison avec des données de référence prédéterminées stockées,

f) utiliser dans ladite étape de dérivation d'information se rapportant à la propriété caractéristique de l'aliment un rapport de deux valeurs détectées de l'impédance ou de la composante de celle-ci à deux fréquences différentes ou une différence ou somme de deux valeurs détectées de l'impédance ou de la composante de celle-ci à deux fréquences différentes ou un rapport d'une différence et de la somme de deux valeurs détectées de l'impédance ou de la composante de celle-ci à deux fréquences différentes,

g) fournir ladite information se rapportant à la propriété caractéristique de l'aliment à une interface utilisateur et/ou à une unité d'émission d'informations et/ou une unité de commande pour commander le processus de cuisson.

**2.** Procédé selon la revendication 1, comprenant l'étape suivante : dériver de ladite impédance détectée ou de la composante de celle-ci des informations se rapportant au moins à une autre propriété caractéristique de l'aliment, qui sont au moins un parmi l'âge ou la fraîcheur de l'aliment (20), la teneur en graisses de l'aliment (20) et si l'aliment (20) a été congelé avant ou non.

**3.** Procédé selon la revendication 2, comprenant au moins une combinaison arbitraire des étapes ou propriétés suivantes :

a) dériver les information se rapportant au type de l'aliment, incluant en particulier différents types de viande et/ou de poisson et/ou de légumes, à partir de ladite impédance détectée ou de la composante de celle-ci avant ladite étape consistant à obtenir des informations se rapportant à l'âge ou à la fraîcheur de l'aliment et/ou sur la base des mêmes valeurs d'impédance détectées ou de la composante de celle-ci que ladite étape d'obtention des informations se rapportant à l'âge ou à la fraîcheur de l'aliment,
b) utiliser ladite information se rapportant à la propriété caractéristique de l'aliment pour adapter le temps de cuisson et/ou la température de cuisson du processus de cuisson en fonction de cette information, où en particulier pour un aliment moins frais ou âgé et/ou pour un aliment déjà congelé avant, le temps de cuisson est sélectionné pour qu'il soit plus long et/ou la température de cuisson est sélectionnée pour qu'elle soit plus élevée ou pour qu'elle soit à un niveau élevé pendant un temps plus long,

**4.** Procédé selon l'une des revendications 1 à 3, dans lequel les données de référence peuvent être complétées par l'utilisateur.

**5.** Procédé selon l'une des revendications 1 à 4, comprenant au moins une ou bien une combinaison arbitraire des étapes ou propriétés suivantes :

a) ladite étape de détection de l'impédance électrique (Z) ou d'au moins une composante précitée de celle-ci de l'aliment pour une obtention suivante d'information se rapportant à la propriété caractéristique de l'aliment a lieu avant ou au début du processus de cuisson et/ou dans une plage de température prédéfinie, de préférence à une température basse, par exemple une température ambiante,
b) dans ladite étape de détection de l'impédance électrique (Z) ou d'au moins une composante précitée de celle-ci de l'aliment, plusieurs valeurs sont mesurées dans un intervalle de temps prédéfini, par exemple, plusieurs minutes, à des instants prédéterminés, par exemple toutes les 10 à 60 secondes.

**6.** Procédé selon l'une des revendication 1 à 5,

a) dans lequel pour n > 1 fréquences m étapes de mesure sont exécutées avec $0 < m \leq n$, durant chacune desquelles une tension est appliquée, où chacune des fréquences n se trouve dans au moins une des tensions appliquées et/ou
b) où une des deux fréquences différentes (f) est sélectionnée pour qu'elle soit de 50 kHz, et une autre des deux fréquences différentes (f) est sélectionnée pour qu'elle soit de 5 kHz.

**7.** Procédé selon la revendication 6, comprenant au moins une ou une combinaison arbitraire des étapes ou propriétés suivantes :

a) comparer les valeurs détectées de l'impédance ou de la composante de celle-ci à une, deux ou plusieurs fréquences avec le même nombre de valeurs de référence stockées pour les propriétés caractéristiques de l'aliment déterminées préalablement aux mêmes fréquences et déterminer la propriété caractéristique de l'aliment en déterminant le degré le plus élevé de coïncidence avec les valeurs de référence, par exemple par une norme mathématique, par exemple la norme Euclidien, ou métrique,
b) utiliser dans ladite étape d'obtention d'informations se rapportant à la propriété caractéristique de l'aliment une fonction ou algorithme de calcul des valeurs détectées de l'impédance ou de la composante de celle-ci à une, deux ou plusieurs fréquences.

**8.** Procédé selon l'une des revendications 1 à 7, comprenant au moins une ou une combinaison arbitraire des étapes ou propriétés suivantes :

a) utiliser dans ladite étape d'obtention des informations se rapportant à la propriété caractéristique, en particulier l'âge ou la fraîcheur et/ou le type de l'aliment la résistance ohmique (R) comme composante de l'impédance,

b) la résistance ohmique (R) sur le même spectre de fréquences pour différentes propriétés caractéristiques, en particulier pour différents types d'aliment ainsi que pour des âges d'aliment différents, diffère en valeurs absolues ainsi que dans la première dérivée et la seconde dérivée,

c) à des fréquences plus basses, la résistance ohmique (R) de l'aliment frais, en particulier de la viande comme de la volaille, est plus élevée que celle d'un aliment âgé et/ou dans lequel la résistance ohmique (R) de l'aliment frais diminue plus fortement avec une fréquence qui augmente que celle d'un aliment âgé et/ou dans lequel, à des fréquences élevées (f), la résistance ohmique (R) de l'aliment frais est plus élevée que celle d'un aliment âgé et/ou dans lequel la courbe de la résistance ohmique pour un aliment frais présente un point tournant en contraste avec un aliment âgé.

9. Procédé selon l'une des revendications 1 à 8, comprenant au moins une ou une combinaison arbitraire des étapes ou propriétés suivantes :

a) utiliser dans ladite étape d'obtention des informations se rapportant à la propriété caractéristique de l'aliment la réactance (X) comme la composante de l'impédance ,

b) la réactance sur le même spectre de fréquences pour différentes propriétés caractéristiques, en particulier pour différents types d'aliment ainsi que pour un âge d'aliment différent, diffère en valeurs absolues ainsi que dans la première dérivée et la deuxième dérivée,

c) la réactance d'un aliment frais, en particulier de la viande comme de la volaille, ainsi que d'un aliment âgé présente un minimum dans un spectre de fréquences donné, et la réactance (X) de l'aliment frais au minimum est plus petite que la réactance (X) de l'aliment âgé au minimum.

10. Procédé selon l'une des revendications 1 à 9, comprenant au moins une ou une combinaison arbitraire des étapes ou propriétés suivantes :

a) utiliser dans ladite étape d'obtention des informations se rapportant à une propriété caractéristique de l'aliment l'angle de phase (φ) comme la composante de l'impédance,

b) l'angle de phase (φ) sur le même spectre de fréquences pour des propriétés caractéristiques différentes, en particulier pour des aliments différents ainsi que pour un âge d'aliment différent, diffère en valeurs absolues ainsi que dans la première dérivée et la deuxième dérivée,

c) l'angle de phase (φ) de l'aliment frais, en particulier de la viande comme de la volaille, a un minimum dans un spectre de fréquences donné, tandis que l'angle de phase (φ) de l'aliment âgé n'a pas de minimum dans ce spectre de fréquences donné,

d) le rapport des angles de phase (φ) à deux fréquences différentes (f) diminue au fur et à mesure qu'augmente l'âge de l'aliment et est en particulier utilisé pour vérifier la qualité de l'aliment avant ou au début du processus de cuisson,

e) l'angle de phase présente pour des légumes un maximum bien défini dans un spectre de fréquences donné mais à une fréquence différente pour différents légumes ainsi qu'une valeur absolue différente,

f) l'angle de phase a pour de la viande différente des valeurs absolues aux mêmes fréquences.

11. Procédé selon l'une quelconque des revendications 1 à 10, comprenant au moins une ou une combinaison arbitraire des étapes ou propriétés suivantes ;

a) obtenir à partir de ladite impédance détectée ou de la composante de celle-ci des informations se rapportant à la propriété caractéristique de l'aliment en calculant un ou plusieurs paramètres électriques (R ; X ; φ) de l'aliment (20) à partir de l'impédance électrique (Z) ou d'une composante de celle-ci et comparer lesdits paramètres électriques (R ; X ; φ) avec une base de données,

b) détecter, lors d'une autre étape durant le processus de cuisson et/ou lorsque l'aliment a été soumis à la cuisson déjà, l'impédance électrique (Z) ou au moins une composante précitée de celle-ci de l'aliment à nouveau pour déterminer la progression de la cuisson et/ou la température de la cuisson dans la région de l'aliment,

c) où en particulier un rapport des angles de phase (φ) à deux fréquences différentes (f) est calculé comme une fonction de la température (T) de l'aliment (20),

d) obtenir en outre de ou comme l'information se rapportant à l'âge ou la fraicheur de l'alimentation une information se rapportant à la quantité ou concentration estimée de bactéries dans l'aliment.

12. Sonde d'aliment (10) pour l'insertion dans un aliment (20), réalisée pour l'utilisation dans le procédé selon l'une quelconque des revendications 1 à 11 et comprenant :

- une tige oblongue (12) réalisée en un matériau non conducteur,
- une portion frontale de la tige oblongue (12), ladite portion frontale est réalisée pour l'entrée dans l'aliment (20),
- une première électrode (14) réalisée en un matériau conducteur et agencée à la portion frontale de la tige (12),
- une deuxième électrode (16) réalisée en un matériau conducteur et agencée à la portion frontale de la tige (12) à une distance prédéterminée de la première électrode (14), et
- une pointe (18) agencée à l'extrémité frontale de la tige (12), où
- la première électrode (14) et la deuxième électrode (16) sont agencées en série le long de l'axe longitudinal de la tige (12),
- la sonde d'aliment (10) est connectée ou peut être connectée à une alimentation de tension et un circuit de commande d'un four de cuisson ou d'un appareil de cuisson, et
- une tension peut être appliquée entre la première électrode (14) et la deuxième électrode (16) de sorte qu'une tension électrique (22) est produite entre et dans l'environnement de la première électrode (14) et de la deuxième électrode (16).

FIG 1

FIG 2

EP 2 500 724 B1

FIG 3

FIG 4

FIG 5

## FIG 6

$\phi\ [°]$

46

44

f

## FIG 7

$\dfrac{\phi\ (50\ kHz)}{\phi\ (5\ kHz)}$

48

0   1   2   3   4   5   6

t [d]

## FIG 8

**EP 2 500 724 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20060174775 A1
- DE 3621999 A1
- EP 0375366 A